# EUROPÄISCHE PATENTSCHRIFT

(11) **EP 2 026 111 B1**
(45) Veröffentlichungstag und Bekanntmachung des Hinweises auf die Patenterteilung: **12.05.2010**
(21) Anmeldenummer: 07015830.8
(22) Anmeldetag: 10.08.2007
(51) Int. Cl.: G02B 7/00

(54) **Steuerung für den Antrieb eines Operationsmikroskops**
Controller for operating an operation microscope
Commande d'entraînement d'un microscope d'opération

(43) Veröffentlichungstag der Anmeldung: 18.02.2009
(73) Patentinhaber: Möller-Wedel GmbH, 22880 Wedel (DE)
(72) Erfinder: Schmidt, Martin, 23611 Bad Schwartau (DE)
(74) Vertreter: Glawe, Delfs, Moll

(56) Entgegenhaltungen:
- EP-A- 1 400 828
- EP-A- 1 804 151
- DE-A1- 19 640 907
- US-A- 4 912 388
- US-A- 4 989 253

## Beschreibung

Die Erfindung betrifft ein Operationsmikroskop mit einem OP-Sauger und mit einem Antrieb, der einen Motor für seitliche Verschwenkung des Mikroskops und einen Motor für eine Vorwärts-Rückwärts-Bewegung des Mikroskops in einer im wesentlichen zur Beobachtungsrichtung senkrechten Richtung aufweist, mit Schaltelementen zum Steuern der Motoren.

Bei bildgebenden medizinischen Geräten ist es typisch, eine Handjustierung vorzusehen, mit der das Gerät manuell nachpositioniert werden kann. Diese Positionierung ist oft aufwändig, da vielfach große Massen zu bewegen sind. Weiterhin wird es beim Neupositionieren notwendig, das betreffende Gerät zu berühren. Dadurch ergeben sich bei intra-operativ benutzten Geräten Sterilitätsprobleme. Ein drittes Problem ist der oft unverhältnismäßige zeitliche Aufwand für die Neupositionierung. Es ist nämlich i.A. bei einer Neupositionierung erforderlich, die gerade benutzten OP-Instrumente abzugeben und hinterher wieder aufzunehmen. Neben zusätzlichen Sterilitätsproblemen entsteht auch ein erheblicher Zeitverlust, der intra-operativ (wegen Blutsperre, etc.) sehr problematisch sein kann. Dadurch wird eine Neupositionierung beispielsweise bei Mikroskopen häufig nicht durchgeführt, obwohl sie eigentlich für den OP-Ablauf notwendig wäre. Weiterhin ergibt sich bei Mikroskopen das Problem, nach einer Neupositionierung auch eine Neufokussierung durchzuführen.

Es ist möglich, alle sechs Gelenkachsen eines Mikroskops, welches zurzeit im Handbetrieb bewegt wird, mit Motoren und Getrieben auszurüsten. Hierbei entstehen allerdings hohe Kosten (für jede der Achsen sind Motoren und hochwertige Getriebe erforderlich), und eine ergonomische Art der Ansteuerung von komplexen Gelenkmechanismen dieser Art ist nicht bekannt.

Derzeit existieren Mikroskope, die über einen Servo-Antrieb der eingangs genannten Art verfügen. Dabei wird über zwei Motoren sowohl eine seitliche Verschwenkung als auch eine Vor-Zurück-Bewegung in horizontaler Richtung ermöglicht. In zwei Handgriffen (seitlich am Mikroskop angebracht) sind dabei Kraft-Moment-Sensoren integriert, die den Bewegungsdruck und die gewünschte Bewegungsrichtung erfassen. Auf Druck gegen die Handgriffe wird nun das Mikroskop entsprechend seitlich verschwenkt oder vorwärts/rückwärts verschoben.

Damit werden jedoch weder die angesprochenen Sterilitätsprobleme noch das Problem des hohen Zeitbedarfs bei der Neupositionierung gelöst. Weiterhin ergibt sich die Anforderung, das Mikroskop in einfacher und ergonomischer Weise seitlich verschwenken zu können, falls weitere bildgebende Instrumente in das Mikroskop integriert sind.

Ein weiteres Problem der Steuerung des Operationsmikroskops ergibt sich, wenn MR- oder CT-Daten in das Sichtfenster des Mikroskops eingespiegelt werden sollen. Dies ist aus Gründen der genauen Navigation häufig sehr wünschenswert. Nach einer Registrierung von prä-operativ aufgenommenen MR- oder CT-Daten ergibt sich üblicherweise ein Fehler, der zum Beispiel durch den sogenannten Brain-Shift (Verschiebung und Verformung des Gewebes nach Öffnung der Schädeldecke) entsteht.

Ein Operationsmikroskop mit einem sterilisierbaren Handshalter ist aus der Druckschrift EP 1400 828 A1 bekannt.

Es ist Aufgabe der Erfindung, ein Operationsmikroskop der eingangs genannten Art zu schaffen, mit der die Positionierung schneller und einfacher in ergonomischer Weise während der Operation durchgeführt werden kann, insbesondere ohne die Instrumente dazu abgeben zu müssen.

Weitere Ziele der Erfindung sind es, durch besondere Ausgestaltung des Operationsmikroskops auch weitere oben genannte Vorgänge beeinflussen zu können.

Die erfindungsgemäße Lösung besteht darin, dass des Operationsmikroskop eine Steuereinheit mit vier kreuzförmig angeordneten Schaltelementen aufweist, die an dem OP-Sauger angebracht ist, wobei die in Längsrichtung des OP-Saugers vorderen und hinteren Schaltelemente den Motor für die Vorwärts-RückwärtsBewegung steuern und die seitlichen Schaltelemente den Motor für die seitliche Verschwenkung steuern.

Vorteilhafte Ausführungsform des erfindungsgemäßen Operationsmikroskops, das auch eine motorisch einstellbare Vergrößerung, motorisch oder elektrisch verstellbare Beleuchtungsintensität und/oder motorisch oder elektrisch verstellbare Beleuchtungsart haben, zeichnen sich dadurch aus, dass die entsprechende Einstellung ebenfalls mit der Steuereinheit vorgenommen werden kann.

So werden beispielsweise in der Neurochirurgie in Verbindung mit konventionellen Operationsmikroskopen moderne fluoreszenztechnische Methoden zur Markierung eingesetzt (z.B. die sogenannte ALA-Fluoreszenzmethode). Bei diesem Verfahren nimmt der Patient vor der Operation ein Pharmakon ein, dessen Stoffwechselprodukt sich in bestimmten Gehirntumoren anreichert. Wird dieses Tumorgewebe nun mit geeignetem Licht bestrahlt, zum Beispiel im tiefblauen, violetten bis nah ultravioletten Spektralbereich (im Weiteren "Blaulicht" genannt), fluoreszieren derartige Tumoren intensiv rötlich und sind wesentlich besser von umgebendem, gesundem Gewebe zu unterscheiden als unter konventioneller (Weißlicht-) Mikroskopbeleuchtung.

Dieser Effekt wird dadurch noch intensiviert, dass im optischen Strahlengang des Mikroskops ein Analyse-Filter eingeschwenkt wird, das auch vom Gewebe zurück gestreutes Blaulicht fast vollständig herausfiltert, während es die mögliche Tumorfluoreszenz durchlässt. Nur ein geringer Anteil von Blaulicht wird vom Filter durchgelassen, um dem Operateur auch bei eingeschwenktem Analyse-Filter eine Orientierungsmöglichkeit zu geben. Allerdings kann sich der Chirurg bei konventioneller Weißlicht-Mikroskopbeleuchtung besser orientieren und das zum Tumor umgebende Gewebe besser beurteilen als bei Beleuchtung mit Blaulicht, so dass die Anforderung besteht, zur Nutzung der Stärken beider Beleuchtungsarten während der Operation schnell zwischen diesen umschalten zu können.

Erfindungsgemäß ist die Steuereinheit an dem OP-Sauger angebracht. Der OP-Sauger, mit dem während der Operation Blut und andere Körperflüssigkeiten abgesaugt werden, ist über einen Schlauch ohnehin mit dem unsterilen Bereich des OP verbunden. Daher kann die angebrachte Steuereinheit, die im Folgenden wegen ihrer besonderen Form auch als Kreuzschalter bezeichnet wird, problemlos und kostengünstig auch mit dem unsterilen Bereich verkabelt werden.

Die Schaltelemente können Tasten oder Schalter seien.

Die Steuereinheit beziehungsweise der Kreuzschalter erlauben eine Angabe von vier Raumrichtungen. Die jeweils gewählte Richtung ist intuitiv erkennbar. Der Hauptvorteil dieser Anordnung ist ein ergonomischer: Die Richtung der gewählten Bewegung ist nämlich auch dann intuitiv erkennbar, wenn weder der Sauger noch das Mikroskop sich räumlich in einer Standardstellung befinden. Wesentlich ist, dass der Chirurg zum Zeitpunkt, in dem die entsprechende Taste oder der entsprechende Schalter betätigt wird, durch das Mikroskop schaut und dadurch die Raumrichtung, in die jeweils bewegt wird, intuitiv festlegbar ist: Das Mikroskop kann nämlich beispielsweise immer vom Chirurgen aus gesehen nach rechts bewegt werden, wenn der Chirurg die rechte Taste des Kreuzschalters betätigt. Dadurch können Missverständnisse ausgeschlossen werden, auch dann, wenn der Sauger gerade nicht in Standardrichtung zum Mikroskop ausgerichtet ist.

Dies erstaunt natürlich, da die Steuereinheit keine richtungsempfindlichen Sensoren aufweist und nicht "weiß", wie das Mikroskop ausgerichtet ist. Es ist das Verdienst der Erfindung, erkannt zu haben, dass der Operateur, wenn er den Patienten aus einer anderen Richtung operiert, nicht nur die Längsachse des chirurgischen Hilfsinstruments neu ausrichtet, sondern auch das Mikroskop mit den Antrieben für die beiden Richtungen, in der es verstellt werden kann. Die Betätigung "nach vorne" bewirkt also immer auch eine Verstellung des Bildfeldes "nach vorne". Entsprechendes gilt für die anderen Richtungen.

Diese Art des intuitiven Erkennens der jeweils gewünschten Richtung ist mit anderen Anordnungen für den Schalter (z.B. Fußschalter) kaum zu erreichen.

Da bei dieser Anordnung nur der Mikroskopkopf (über die beiden vorhandenen Motoren) verschwenkt wird, und nicht das gesamte Mikroskop über 6 Achsen, ergibt sich zunächst die Schwierigkeit, den Fokus nachzujustieren, wenn neu positioniert wurde.

Bei einer vorteilhaften Ausführungsform ist vorgesehen, dass die Steuereinheit in der Mitte zwischen den vier Schaltelementen einen zusätzlichen Schalter zum Wechseln zwischen Betriebsarten aufweist. Insbesondere kann bei einem Mikroskop mit einem Antrieb, der einen weiteren Motor für die Fokussierung des Mikroskops aufweist, vorgesehen sein, dass die weitere Betriebsart Fokussierung des Mikroskops ist, die über zwei gegenüber angeordnete Schaltelemente der Steuereinheit steuerbar ist.

Wenn der Chirurg den in der Mitte angeordneten Schalter betätigt, wird bei dieser Ausführungsform zwischen Positioniermodus und Fokussiermodus hin- und hergeschaltet. Anschließend dienen zwei der vier kreuzweise angeordneten Schaltelemente am Kreuzschalter zum Fokussieren in den beiden Richtungen.

Eine weitere bevorzugte Ausführungsart sieht vor, die Fokussierung zu automatisieren, und zwar in der folgenden Weise: Wird angenommen, dass das Zielgebiet eben ist, und sind zwei Fokuseinstellungen aus vorherigen Positionen bekannt, so kann eine dritte, und jede weitere Fokussierung automatisch durch Triangulation berechnet werden.

Bei einer bevorzugten Ausführungsform (falls der Sauger kein Einwegsauger ist) wird der Kreuzschalter als Einweg- oder Mehrwegprodukt (aufsteckbar) realisiert. Zum Aufstecken kann ein Klemmmechanismus verwendet werden. Bei einer weiteren vorteilhaften Ausführungsform ist vorgesehen, die Steuereinheit direkt in den Einwegsauger zu integrieren und mit einer Schutzschicht (z.B. aus Kunststoff) zu überziehen.

Soll eine vorhandene Einrichtung des Operationsmikroskop zur Beleuchtung mit Blaulicht gesteuert werden, kann die erfindungsgemäße Steuereinheit in einfacher Weise benutzt werden. Mit dem Schalter zum Wechseln der Betriebsart wird die Steuerung in einen hierfür vorgesehenen Modus umgeschaltet. Der Chirurg kann dann während der Resektion eines Tumors durch schnelles Schalten zwischen konventioneller Weißlicht- einerseits und Fluoreszenzbeleuchtung andererseits, beispielsweise durch sequenzielle Drücken einer der Tasten eine gute Sichtbarkeit des Tumors durch die Bestrahlung mit Blaulicht mit einer guten Übersicht über das OP-Gebiet und eine farblich korrekte Wiedergabe des Gewebes bei Weißlichtbeleuchtung kombinieren.

Bei einer weiteren bevorzugten Ausführungsform wird zusätzlich die Einspiegelung von MR- oder CT-Daten in das Mikroskop ermöglicht. Wird der mittlere Schalter zum Beispiel länger betätigt, so kann der Steuereinheit dadurch mitgeteilt werden, dass eine translatorische Bewegung des eingespiegelten MR- oder CT-Bildes relativ zur tatsächlich sichtbaren Anatomie beabsichtigt ist. Die Notwendigkeit der Verschiebung kann sich z.B. im OP-Verlauf ergeben, wenn klar wird, dass durch den Brain-Shift die initiale Registrierung zwischen Anatomie und MR- bzw. CT-Daten fehlerhaft ist. Über den Kreuzschalter wird dann eine Bewegung des eingespiegelten Bildes durchgeführt. Auch eine rotatorische Bewegung des Bildes ist erfindungsgemäß möglich, indem ein Rotationszentrum geeignet z.B. durch einen Laserpointer in der Mikroskopoptik markiert wird, das gewünschte neue Rotationszentrum dann durch Verschiebungsbewegung via Kreuzschalter angefahren wird, und danach durch Aktivieren der Schalter die Drehbewegung im Uhrzeigersinn (z.B. rechter Schalter) oder Gegenuhrzeigersinn (linker Schalter) angestoßen wird. Diese manuelle Feinregistrierung erfordert die Identifikation geeigneter Landmarken. Hierfür kann ein geeignetes separates Software-Trainingssystem von Nutzen sein. Das Trainingssystem trainiert die manuelle Feinregistrierung an Hand von festen, hochgenau vorregistrierten Datensätzen.

Bei einer weiteren bevorzugten Ausführungsform wird auf dem Sauger zusätzlich ein Marker für ein video-optisches Trackingsystem aufgebracht. Dadurch wird es möglich, Zielrichtungen für den Blickwinkel des Mikroskops direkt durch den Sauger vorzugeben. Nachdem der Sauger in die gewünschte Blickrichtung gebracht wurde, wird der Kreuzschalter benutzt, um die eigentliche Bewegung freizugeben. Hierbei kann es erforderlich sein, die Bewegung in mehrere Einzelbewegungen mit Links-Rechts- bzw. Vor-Zurückkomponente zu zerlegen, oder auch eine Pivotbewegung um einen festen Punkt im Gewebe auszuführen. Die Pivotbewegung bezieht sich dann auf den von der Saugerspitze angezeigten Punkt in der Anatomie.

Die Erfindung wird im Folgenden an Hand von vorteilhaften Ausführungsformen unter Bezugnahme auf die beigefügten Zeichnungen beispielsweise beschrieben. Es zeigen:
- Fig. 1: in Draufsicht das Äußere einer erfindungsgemäßen Steuereinheit;
- Fig. 2: ein chirurgisches Hilfsinstrument mit daran ange- brachter erfindungsgemäßen Steuereinheit;
- Fig. 3: ein Operationsmikroskop mit einem Antrieb für die Stellung des Mikroskops und einer erfindungsgemäßen Steuereinheit, die an einem chirurgischen Hilfsin- strument angebracht ist; und
- Fig. 4: ein Schema zur Erläuterung der automatischen Fokus- sierung.

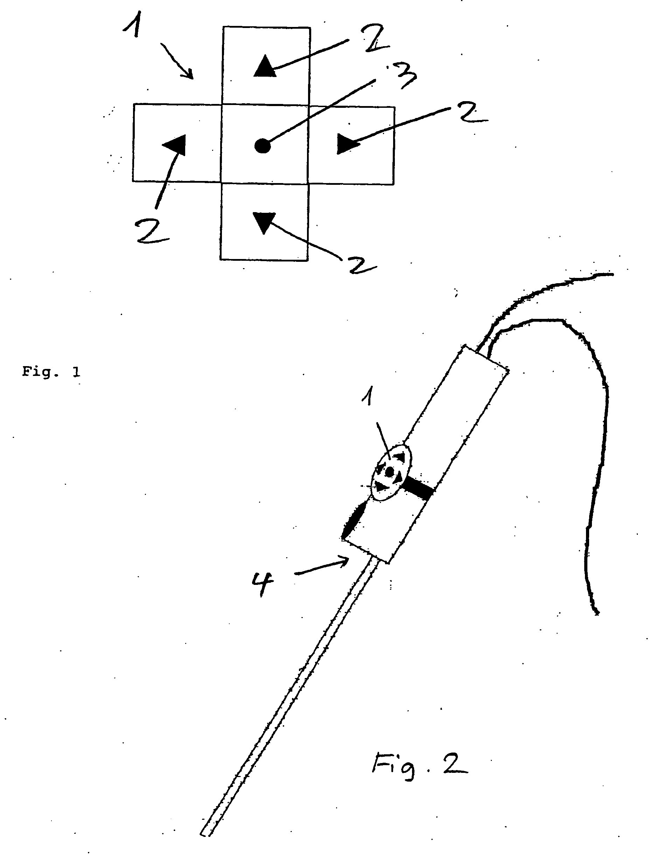

In Fig. 1 ist in Draufsicht das Äußere einer erfindungsgemäßen Steuereinheit 1 mit vier Tasten oder Schaltern 2 für die entsprechenden Bewegungen des Mikroskops und für die anderen genannten Zwecke dargestellt. Die Tasten oder Schalter 2 sind bei dieser Ausführungsform mit Richtungspfeilen versehen. In der Mitte zwischen den Tasten oder Schaltern 2 ist die Umschalttaste 3 für die Umschaltung auf andere Betriebsarten gezeigt.

In Fig. 2 ist ein chirurgisches Hilfsinstrument 4 gezeigt, das in diesem Falle ein OP-Sauger ist und an dem die erfindungsgemäße Steuereinheit 1 angebracht ist. Die Richtung nach vorwärts oder rückwärts wird dabei durch die Längsachse des Hilfsinstrument 4 definiert.

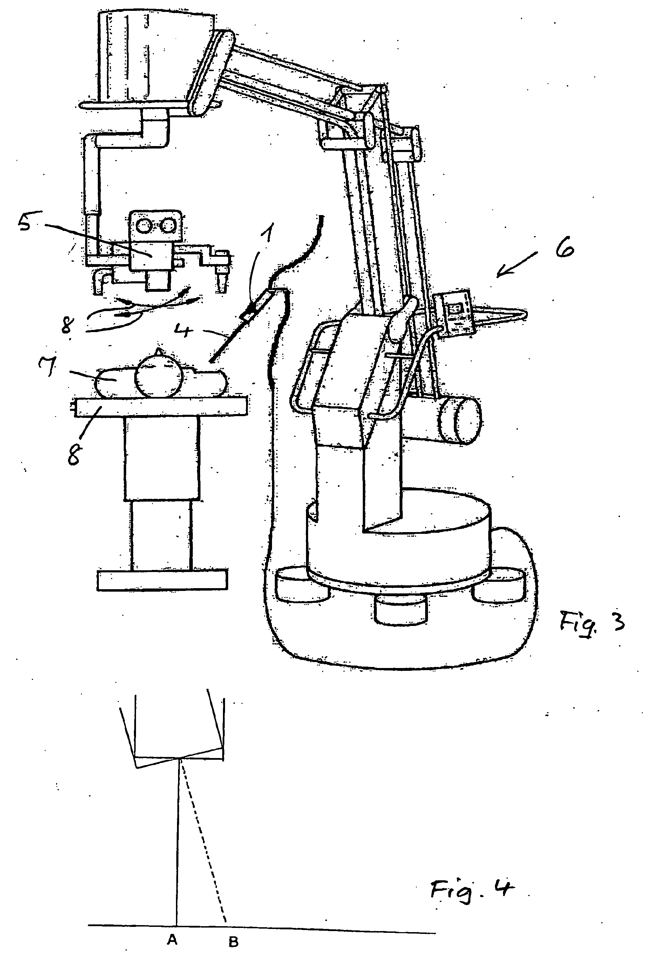

In Fig. 3 ist ein Operationsmikroskop 5 mit einem motorisch vorstellbaren Stativ 6 dargestellt. Der Patient 7 befindet sich auf einem Operationstisch 8. Mit dem OP-Sauger 4 können während der Operation Flüssigkeiten abgesaugt werden. Mithilfe der Steuereinheit 1, die am OP-Sauger 4 angebracht ist, kann das Mikroskop 5 in Richtung der Pfeile 8 verstellt werden.

Fig. 4 erläutert das Prinzip der automatischen Fokussierung. Mit 5 ist dabei das Mikroskop beziehungsweise sein Objektiv angedeutet. Beispielsweise seien die beiden Fokuseinstellungen, die zu den Punkten A und B auf der Zentralachse des Mikroskops gehören, bekannt. Für eine dritte Position ist damit die korrekte Fokuseinstellung direkt aus dem Schwenkwinkel und aus der Steuerung bekannten Gelenkparametern berechenbar.

## Patentansprüche

1. Operationsmikroskop (5) mit einem OP-Sauger und mit einem Antrieb, der einen Motor für seitliche Verschwenkung des Mikroskops (5) und einen Motor für eine Vorwärts-Rückwärts-Bewegung des Mikroskops (5) in einer im wesentlichen zur Beobachtungsrichtung senkrechten Richtung aufweist, und mit einer Steuerung für den Antrieb mit Schaltelementen (2) zum Steuern der Motoren, **dadurch gekennzeichnet, dass** sie eine Steuereinheit (1) mit vier kreuzförmig angeordneten Schaltelementen (2) aufweist, wobei die in Längsrichtung vorderen und hinteren Schaltelemente (2) den Motor für die Vorwärts-Rückwärts-Bewegung steuern und die seitlichen Schaltelemente (2) den Motor für die seitliche Verschwenkung steuern und
dass die Steuereinheit (1) an dem OP-Sauger (4) angebracht ist.

2. Operationsmikroskop nach Anspruch 1, **dadurch gekennzeichnet, dass** die Schaltelemente (2) Tasten sind.

3. Operationsmikroskop nach Anspruch 1, **dadurch gekennzeichnet, dass** die Schaltelemente (2) Schalter sind.

4. Operationsmikroskop nach einem der Ansprüche 1 bis **3**, **dadurch gekennzeichnet, dass** die Steuereinheit (1) in der Mitte zwischen den vier Schaltelementen (2) einen zusätzlichen Schalter (3) zum Wechseln zwischen Betriebsarten aufweist.

5. Operationsmikroskop nach Anspruch **4** mit einem Antrieb, der einen weiteren Motor für die Fokussierung des Mikroskops aufweist, **dadurch gekennzeichnet, dass** die weitere Betriebsart Fokussierung des Mikroskops ist, die über zwei gegenüber angeordnete Schaltelemente (2) der Steuereinheit steuerbar ist.

6. Operationsmikroskop nach Anspruch 5, **dadurch gekennzeichnet, dass** sie eine automatische vom Schwenkwinkel abhängige Fokussierung aufweist.

7. Operationsmikroskop nach einem der Ansprüche 1 bis **6**, **dadurch gekennzeichnet, dass** die Steuereinheit (1) auf dem OP-Sauger (4) aufsteckbar ist.

8. Operationsmikroskop nach einem der Ansprüche 1 bis 6, **dadurch gekennzeichnet, dass** die Steuereinheit (1) in den OP-Sauger (4) integriert ist.

9. Operationsmikroskop nach einem der Ansprüche 1 bis 8, **dadurch gekennzeichnet, dass** die Steuereinheit (1) eine Schutzschicht, insbesondere eine Kunststofffolie aufweist, mit der zumindest die Schaltelementen (2, 3) abgedeckt sind.

10. Operationsmikroskop nach Anspruch 4, **dadurch gekennzeichnet, dass** die Steuereinheit (1) zum Verschieben von in das Mikroskop eingespiegelten Daten, insbesondere MR- oder CT-Daten ausgebildet ist.

11. Operationsmikroskop nach einem der Ansprüche 1 bis **10**, **dadurch gekennzeichnet, dass** dieSteuereinheit (1) fest am chirurgischen Hilfsinstrument (4) angebracht ist.

12. Operationsmikroskop nach Anspruch 11, **dadurch gekennzeichnet, dass** die Steuereinheit (1) vom chirurgischen Hilfsinstrument (4) lösbar ist.

13. Operationsmikroskop nach einem der Ansprüche 1 bis 12, das auch eine motorisch einstellbare Vergrößerung, motorisch oder elektrisch verstellbare Beleuchtungsintensität und/oder motorisch oder elektrisch verstellbare Beleuchtungsart hat, **dadurch gekennzeichnet, dass** die Steuereinheit zum Einstellen dieser Betriebswerte und -größen ausgebildet ist.

## Claims

1. Operating microscope (5) with a surgical suction unit and with a drive, which has a motor for lateral pivoting of the microscope (5) and a motor for forward/backward movement of the microscope (5) in a direction basically perpendicular to the observation direction, and with a control for the drive with switch elements (2) for controlling the motors, **characterized in that** it has a control unit (1) with four switch elements (2) arranged in a cross, in which the front and back switch elements (2) in the longitudinal direction control the motor for the forward/backward movement and the lateral switch elements (2) control the motor for the lateral pivoting and **in that** the control unit (1) is attached to the surgical suction unit (4).

2. Operating microscope according to Claim 1, **characterized in that** the switch elements (2) are pushbuttons.

3. Operating microscope according to Claim 1, **characterized in that** the switch elements (2) are switches.

4. Operating microscope according to one of Claims 1 to 3, **characterized in that** the control unit (1) has an additional switch (3), located in the middle between the four switch elements (2), for switching between methods of operation.

5. Operating microscope according to Claim 4 with a drive, which has a further motor for focussing the microscope, **characterized in that** the further method of operation is focussing the microscope, which can be controlled by two switch elements (2) of the control unit that are arranged opposite to one another.

6. Operating microscope according to Claim 5, **characterized in that** it has automatic focussing that depends on the pivot angle.

7. Operating microscope according to one of Claims 1 to 6, **characterized in that** the control unit (1) can be pushed onto the surgical suction unit (4).

8. Operating microscope according to one of Claims 1 to 6, **characterized in that** the control unit (1) is integrated in the surgical suction unit (4).

9. Operating microscope according to one of Claims 1 to 8, **characterized in that** the control unit (1) has a protective layer, in particular a foil made of plastics, by means of which at least the switch elements (2, 3) are covered.

10. Operating microscope according to Claim 4, **characterized in that** the control unit (1) is designed for displacing data mirrored into the microscope, in particular MR or CT data.

11. Operating microscope according to one of Claims 1 to 10, **characterized in that** the control unit (1) is fixedly attached to the surgical auxiliary instrument (4).

12. Operating microscope according to Claim 11, **characterized in that** the control unit (1) can be detached from the surgical auxiliary instrument (4).

13. Operating microscope according to one of Claims 1 to 12, which also has an enlargement that can be set by motor, an illumination intensity that can be adjusted by motor or electrically and/or a type of illumination that can be adjusted by motor or electrically, **characterized in that** the control unit is designed to set these operating values and variables.

## Revendications

1. Microscope chirurgical (5) avec un aspirateur chirurgical, comportant un système d'entraînement, qui comporte un moteur pour le pivotement latéral du microscope chirurgical (5) et un moteur pour un mouvement vers l'avant et l'arrière du microscope chirurgical (5) dans une direction sensiblement perpendiculaire à la direction d'observation, et comportant une commande pour le système d'entraînement qui possède des éléments de commande (2) pour la commande des moteurs, **caractérisé en ce qu'**il comporte une unité de commande (1) avec quatre éléments de commande (2) disposés en croix, les éléments de commande (2) avant et arrière dans la direction longitudinale commandant le moteur pour le mouvement vers l'avant et l'arrière et les éléments de commande (2) latéraux commandant le moteur pour le pivotement latéral, et **en ce que** l'unité de commande (1) est montée sur l'aspirateur chirurgical (4).

2. Microscope chirurgical selon la revendication 1, **caractérisé en ce que** les éléments de commande (2) sont des touches.

3. Microscope chirurgical selon la revendication 1, **caractérisé en ce que** les éléments de commande (2) sont des commutateurs.

4. Microscope chirurgical selon l'une quelconque des revendications 1 à 3, **caractérisé en ce que** l'unité de commande (1) comporte, au milieu entre les quatre éléments de commande (2), un commutateur (3) supplémentaire pour la permutation entre les modes de fonctionnement.

5. Microscope chirurgical selon la revendication 4, comportant un système d'entraînement qui comporte un moteur supplémentaire pour la focalisation du microscope, **caractérisé en ce que** le mode de fonctionnement supplémentaire est la focalisation du microscope, qui peut être commandée par deux éléments de commande (2), face à face, de l'unité de commande.

6. Microscope chirurgical selon la revendication 5, **caractérisé en ce qu'**il comporte une focalisation automatique en fonction de l'angle de pivotement.

7. Microscope chirurgical selon l'une quelconque des revendications 1 à 6, **caractérisé en ce que** l'unité de commande (1) peut être enfichée sur l'aspirateur chirurgical (4).

8. Microscope chirurgical selon l'une quelconque des revendications 1 à 6, **caractérisé en ce que** l'unité de commande (1) est intégrée dans l'aspirateur chirurgical (4).

9. Microscope chirurgical selon l'une quelconque des revendications 1 à 8, **caractérisé en ce que** l'unité de commande (1) comporte une couche de protection, en particulier une feuille plastique, qui recouvre au moins les éléments de commande (2, 3).

10. Microscope chirurgical selon la revendication 4, **caractérisé en ce que** l'unité de commande (1) est configurée pour transférer des données introduites dans le microscope, en particulier des données MR ou CT.

11. Microscope chirurgical selon l'une quelconque des revendications 1 à 10, **caractérisé en ce que** l'unité de commande (1) est montée de manière fixe sur l'instrument chirurgical (4) auxiliaire.

12. Microscope chirurgical selon la revendication 11, **caractérisé en ce que** l'unité de commande (1) peut être démontée de l'instrument chirurgical (4) auxiliaire.

13. Microscope chirurgical selon l'une quelconque des revendications 1 à 12, lequel possède également un agrandissement réglable par un moteur, une intensité lumineuse réglable par un moteur ou par voie électrique et/ou un type d'éclairage réglable par un moteur ou par voie électrique, **caractérisé en ce que** l'unité de commande est configurée pour régler ces valeurs et grandeurs de service.
